# EUROPÄISCHE PATENTSCHRIFT

(11) **EP 2 060 229 B1**
(45) Veröffentlichungstag und Bekanntmachung des Hinweises auf die Patenterteilung: **15.06.2011**
(21) Anmeldenummer: 08001950.8
(22) Anmeldetag: 01.02.2008
(51) Int. Cl.: A61B 5/103, A63B 22/02

(54) **Anordnung zur Ganganalyse**
Assembly for movement analysis
Dispositif d'analyse de la marche

(30) Priorität: 14.11.2007 DE 102007054365
(43) Veröffentlichungstag der Anmeldung: 20.05.2009
(73) Patentinhaber: Zebris Medical GmbH, 88316 Isny im Allgäu (DE)
(72) Erfinder: Brunner, Wolfgang, 88167 Maierhöfen (DE)
(74) Vertreter: Heinze, Ekkehard

(56) Entgegenhaltungen:
- EP-A- 1 386 642
- EP-A- 1 681 080
- GB-A- 2 431 595
- US-A- 5 299 454
- US-A- 6 033 344
- US-A1- 2003 211 896
- US-A1- 2004 192 511
- US-A1- 2006 247 104

## Beschreibung

Die vorliegende Erfindung betrifft eine Anordnung zur Ganganalyse zu Trainings- oder Rehabilitationszwecken.

Vorrichtungen zur Erfassung von Druck- bzw. Kraftverteilungen sind an sich bekannt, beispielsweise aus der DE 36 42 088 C2 und der DE 25 29 475 C3.

Viele der bekannten Vorrichtungen können als Plattformen zur biomechanischen Ganganalyse eingesetzt werden, bei denen der Gang eines Wirbeltiers, insbesondere eines Menschen, gegebenenfalls aber auch eines Pferdes oder Hundes etc., untersucht und analysiert wird. Hierbei besteht aber der Nachteil, dass immer nur ein einziger Schritt und ein einzelner Abrollvorgang aufgenommen werden kann. Um ein natürliches Gangverhalten zu erhalten, ist es dagegen notwendig, den Gang über eine längere Zeit aufzunehmen.

Es wurden daher auch bereits Vorrichtungen und Verfahren zur Ganganalyse, die sich eines Laufbandes bedienen, vorgeschlagen. Beispielhaft sei hierzu hingewiesen auf die DE 40 27 317 C1 oder die US 6,010,465 A.

Ferner wird in R. Kram und A.J. Powell: "A treadmill-mounted force platform" Appl. Physiol. 67 (4): 1692-1698 (1989) eine Messvorrichtung als bekannt beschrieben, bei der ein Laufband über eine Messplattform bzw. Messfläche gezogen wird und somit eine fortlaufende Erfassung von Kräften möglich wird.

Die erstere dieser Druckschriften beschreibt ein Laufband, welches aus einer Vielzahl von Gliedern aufgebaut ist, von denen jedes eine matrixförmige Anordnung von Druck- bzw. Kraftsensoren umfasst, während die zweite ein Laufband mit unter der Bandoberfläche liegender Messplatte mit einer matrixförmigen Anordnung von Druck- bzw. Kraftsensoren beschreibt. Beide Druckschriften lehren, dass mit der jeweiligen Sensorik eine Auswertungseinheit verbunden ist und die US 6,010,465 beschreibt den Aufbau und die Betriebsweise der Auswertungseinheit, etwa zur Auswertung der Position und eines zugeordneten Kraftwertes beim Auftreten auf das Laufband, beispielsweise zur Bestimmung von Drehmomenten und Lasten auf die Gelenke sowie bestimmten Gangparametern, relativ detailliert.

Mit Verbesserungen dieser bekannten Lösungen hinsichtlich der Ableitung differenzierter medizinischer bzw. sportphysiologischer Aussagen befasst sich die internationale Patentanmeldung PCT/EP2006/01 04 71 der Anmelderin. Diese lehrt insbesondere Mittel und Vorgehensweisen zur genauen und differenzierten Erfassung der tatsächlichen Geschwindigkeit des Laufbandes unter Nutzung der Zeit- und Ortsabhängigkeit von auf diesem beim Gehen oder Laufen eines Probanden aufgenommenen Druckverteilungsbildern.

Bekannt ist auch der Einsatz von Anzeigevorrichtungen, wie Anzeigeschirmen, bei Laufband-Anordnungen.

Aus der EP 1 145 682 A2 sind ein auf der Laufband-Technik aufbauendes Rehabilitationsgerät und -verfahren bekannt, bei dem eine Anpassung der Funktion des Laufbandes an den aktuellen Stand der wiederherzustellenden Geh- bzw. Lauffähigkeit eines Patienten vorgesehen ist. Insbesondere wird die Laufbandgeschwindigkeit an einen persönlichen Schrittzyklus des Nutzers angepasst, und das Gerät soll diesem zugleich ein Feedback geben. In einer speziellen Ausführung ist auch die Erfassung von beim Auftreten ausgeübten Druckkräften und deren Auswertung im Rahmen des Gesamtprogramms vorgesehen. Die Druckschrift beschreibt auch den Einsatz eines Anzeigeschirmes in Verbindung mit einer Eingabetastatur zur Anzeige der auf dem Laufband erzeugten Fußabdrücke und zur Einstellung von Laufbandparametern anhand dieser Anzeige.

Aus der US 6,231,527 B1 ist ebenfalls der Einsatz eines Anzeigeschirmes bei einer Laufbandvorrichtung für den sportmedizinischen bzw. Rehabilitations-Einsatz bekannt, wobei hier die Bilder verschiedener Kameras dargestellt werden können, die den Sportler/Patienten in seinen Bewegungen auf dem Laufband aufnehmen.

Aus der US-A-5 299 454 ist eine Anordnung zur Ganganalyse nach dem Oberbegriff des Anspruchs 1 bekannt. Die EP-A-1 386 642 lehrt eine Laufbandanordnung, die eine Projektionsvorrichtung zur Darstellung virtueller Laufumgebungen in Abhängigkeit von Geräteparametern des Laufbandes umfasst.

Der Erfindung liegt die Aufgabe der Bereitstellung einer weiter verbesserten Vorrichtung der oben skizzierten Art zugrunde, die sich besonders für sportmedizinische oder Rehabilitations-Zwecke eignet. Ein Ziel ist es, das Laufband mit integrierter Druckverteilungssensorik nicht nur als reines Analysegerät zur Stand-, Gang- und Laufanalyse einzusetzen, sondern das System zu einem Trainings-/Therapiegerät weiter zu entwickeln.

Diese Aufgabe wird durch eine Anordnung zur Ganganalyse mit den Merkmalen des Anspruchs 1 gelöst. Zweckmäßige Fortbildungen des Erfindungsgedankens sind jeweils Gegenstand der abhängigen Ansprüche.

Mit der erfindungsgemäßen Vorrichtung ist die Aufnahme des Gangs über eine längere Zeit möglich, da ein Laufbandsystem verwendet wird. Dieses Laufbandsystem umfasst ein Endlosband, das über eine Sensorplattform gezogen wird, die mit einer Vielzahl matrixförmig angeordneter Druck- bzw. Kraftsensoren bestückt ist.

Als wesentliche Aspekte bzw. Ausführungsformen der Erfindung sind folgende zu nennen:

Es wird das bekannte Laufband mit Druckverteilungs-Sensorik zunächst zusätzlich mit einer Recheneinheit und Anzeigemitteln wie z.B. einem Bildschirm und/oder einer Vorrichtung für ein akustisches Feedback ausgestattet. In einer Ausführung wird eine Belastungsanalyse durchgeführt, wobei der Proband/Untersucher über ein Feedbacksignal gewarnt wird, wenn die Gewichtsbelastung ein bestimmtes Maß überschreitet. Hierzu wird der Patient etwa über ein Gewichtsentlastungs- und Gurtsystem aufgehängt.

In einer Vereinfachung der bekannten Druckverteilungs-Matrix kann es hierbei ausreichen, dass diese nur aus einer Matrix von Ein-/Ausschaltern besteht, bei denen nicht analoge Druck-/Kraftwerte, sondern nur zwei Schaltzustände erfasst werden. Ein evtl. variierbarer Schaltwiderstand könnte hierbei zur Durchführung des Anfangs beschriebenen Belastungsfeedback verwendet werden.

In einer weiteren Ausführung kann das System die Belastung auf dem linken und rechten Bein unterscheiden. Dies kann in einer einfachen Ausführung dadurch geschehen, dass das Laufband bzw. die Druckverteilungs-Sensorik örtlich in einen linken und rechten Bereich unterteilt wird. Wird die Belastung auf einem Bein überschritten, so wird dies z.B. über ein akustisches Signal angezeigt. Dadurch kann trainiert werden, ein Bein z.B. nach einer Hüftgelenkoperation nur mit einem bestimmten Prozentsatz des Körpergewichts zu belasten. Dazu ist es sinnvoll, dass der Patient sich über ein Geländer oder eine Armstütze entlasten kann. Um den zugelassenen Anteil der Belastung zu ermitteln, kann vor der Messung entweder das Körpergewicht eingegeben werden, oder es wird das gesamte Körpergewicht zunächst mit der im Laufband befindlichen Sensorik gemessen.

In einer weiteren Ausführungsform werden dem Probanden Aufgaben gestellt, die sich auf sein Gehen/Laufen beziehen und dieses beeinflussen. Zum Beispiel läuft der Proband auf einem simulierten Waldweg und muss am Boden dargestellten Wasserpfützen ausweichen. Tritt er in das "Wasser", so wird dies detektiert und zur Anzeige gebracht. Eine entsprechende Auswertung gibt die Erfolgsquote wieder.

Diese Art der Rückkopplung hat neben dem spielerischen Effekt und dem Effekt zur Verbesserung der Koordination auch den Vorteil, dass verschiedene Variationen des Auftretens mit den Füßen provoziert werden, was zu einer besseren Diagnose und zu besseren Möglichkeiten zur Erstellung von orthopädischen Einlegesohlen führen wird. Das normale Gehen auf dem Laufband führt nämlich zu einem eingeschliffenen stereotypen Gehen, bei dem Gangstörungen u.U. nicht aufgezeigt werden. In einer bevorzugten Ausführungsform wird die Geschwindigkeit des Laufbandes sowie der Neigungswinkel des Bandes über die Recheneinheit gesteuert. Dadurch ergeben sich weitere Provokationsmöglichkeiten, wobei wiederum das veränderte Gang- und Abrollverhalten direkt analysiert werden kann.

In einer weiteren Ausführung ist es vorgesehen, in die oder unter der Lauffläche entweder feste Erhebungen bzw. Vertiefungen einzubauen oder Aktuatoren einzubauen mit denen sich auf der Lauffläche Erhöhungen realisieren lassen. Dies könnte z.B. mit einer Matrix aus luft- oder flüssigkeitsgefüllten Kammern realisiert werden. Diese werden ebenfalls von der Recheneinheit gesteuert und können für Provokationstests verwendet werden.

Bei der ersten Variante ist es insbesondere zweckmäßig, wenn das Endlosband mit gang-wirksam strukturierter Oberfläche eine Positionskodierung zur Positionszuordnung von Oberflächenelementen aufweist, die Verarbeitungseinheit Profilspeichermittel zur Speicherung des Profils der gang-wirksam strukturierten Oberfläche aufweist und ihr ein Positionssignalempfänger zur Adressierung des Profilspeichers zugeordnet ist und in der Verarbeitungseinheit ein Verarbeitungsalgorithmus implementiert ist, mit dem die Druckverteilungsbilder den Oberflächenelementen der Lauffläche zugeordnet werden.

Eine vorteilhafte Ausführung der zweiten Variante zeichnet sich dadurch aus, dass der Anordnung von Aktoren eine Profilsteuereinheit zugeordnet ist, die Steuersignale zur Betätigung der Aktoren zur Ausbildung eines vorbestimmten dynamischen Profils der Lauffläche ausgibt, die Verarbeitungseinheit einen Steuersignalempfänger zum Empfang der durch die Profilsteuereinheit ausgegebenen Steuersignale als Positionszuordnungssignale aufweist und in der Verarbeitungseinheit ein Verarbeitungsalgorithmus implementiert ist, mit dem die Druckverteilungsbilder dem dynamischen Profil der Lauffläche zugeordnet werden.

Als konkrete konstruktive Mittel zur Verformung der Bandoberfläche können an sich bekannte mechanische, elektromechanische, hydraulische oder pneumatische Konstruktionen eingesetzt werden, die insbesondere über elektrische Steuersignale mit ausreichender Ansprechgeschwindigkeit angesteuert werden können. Beispiele hierfür sind, dass die Anordnung von Aktoren eine Vielzahl von einzeln angetriebenen Stößeln zur Erzeugung von Erhebungen auf der Lauffläche aufweist, oder dass im Endlosband oder unterhalb der Lauffläche eine Anordnung von jeweils einen steuerbaren Fluidanschluss aufweisenden, durch Fluiddruck elastisch aufweitbaren Kammern zur Ausbildung von Erhebungen auf der Lauffläche vorgesehen ist.

Ein relativ unabhängiger Grundgedanke der Erfindung besteht darin, eine an sich bekannte Laufbandanordnung mit folgenden zusätzlichen Komponenten auszustatten: einer dem Laufband zugeordneten, im Sichtfeld des Probanden angeordneten Bildanzeigefläche, einem Film- oder Video-Wiedergabegerät mit in einem Bildmaterialspeicher gespeichertem Bildmaterial oder einem Anschluss zur Verbindung mit einer Bildmaterial-Datenbasis zum Empfang von Bildmaterial zur Simulation einer Laufumgebung und/oder zur bildlichen Darstellung von Anforderungen, Fragen oder dergleichen an den Probanden sowie einer Synchronisationsstufe zur Synchronisation der Verarbeitung der Druckverteilungsbilder mit den bildlichen Darstellungen auf der Bildanzeigefläche.

Die Realisierung der Bildanzeigefläche, in Verbindung mit dem Film- oder Video-Wiedergabegerät, kann mit einer Vielzahl an sich bekannter und kommerziell verfügbarer Komponenten erfolgen, wobei Zweck und Ausstattungsniveau der vorgeschlagenen Anordnung eine erhebliche Rolle spielen. In einer einfachen Ausführung ist das Film- oder Video-Wiedergabegerät durch einen PC oder ein Fernsehgerät gebildet, dessen Bildschirm als Bildanzeigefläche dient. Eine aufwändigere Ausführung, die aber eine realitätsnähere Simulation natürlicher Laufumgebungen ermöglicht, zeichnet sich dadurch aus, dass das Film- oder Video-Wiedergabegerät einen Laserbeamer aufweist und die Bildanzeigefläche als Projektionsfläche ausgebildet ist.

Beide grundlegenden Realisierungen des Erfindungsgedankens lassen sich zweckmäßig dahingehend ausbauen, dass auch ansteigenden oder abschüssige Laufstrecken simuliert werden. Sie weisen hierzu Neigungseinstellmittel zur Neigungseinstellung des Laufbandes auf, und bei der Realisierung mit optischer Simulation einer natürlichen Laufumgebung wird entsprechendes Bildmaterial erzeugt und in Abstimmung auf die Neigungseinstellung des Laufbandes eingespielt.

Grundsätzlich analog hierzu sind in einer weiteren Ausführung Geschwindigkeitseinstellmittel zur Geschwindigkeitseinstellung des Laufbandes vorgesehen, und auch hierbei kann die dem Probanden gelieferte Simulation auf die aktuelle Geschwindigkeit des Laufbandes abgestimmt sein. Beide Einstellmöglichkeiten können auch sinnvoll kombiniert sein, und insbesondere ist vorgesehen, dass die Synchronisationsstufe mit sich aus dem Bildmaterial oder Anforderungen, Fragen oder dergleichen an den Probanden ergebenden Daten programmierbar ist, derart, dass die Verarbeitung der Druckverteilungsbilder anhand entsprechender Vorgaben erfolgen kann. So kann beispielsweise in der bildlichen Darstellung der Proband angewiesen werden, bestimmte Bereiche der Laufstrecke (d.h. des Laufbandes) nicht zu betreten, und bei einer hierauf abgestimmten Auswertung der Druckverteilungsbilder wird überprüft, ob ihm dies anforderungsgemäß gelungen ist.

In einer weiteren Ausführungsform der Erfindung ist die Anordnung mit zusätzlichen Messmitteln zur Erfassung mindestens einer zweiten biometrischen oder medizinischen Messgröße ausgestattet, wobei die Verarbeitungseinheit zur kombinierten Verarbeitung der Druckverteilungsbilder und von Messwerten der weiteren biometrischen Messgröße ausgebildet ist. Zweckmäßigerweise ist dann außerdem eine Synchronisationseinheit zur Synchronisation der Druck/Kraftverteilungsmessung mit der Erfassung der oder einer weiteren biometrischen bzw. medizinischen Messgröße durch die zusätzlichen Messmittel vorgesehen. In einer Ausgestaltung dieser Ausführung ist zudem die Synchronisationseinheit eingangsseitig mit der Auswertungs- oder Verarbeitungseinheit verbunden, derart, dass Synchronisationssignale im Ansprechen auf Merkmale der Druckverteilungen oder auf die daraus abgeleiteten Werte bzw. Signale ausgegeben werden.

Eine weitere sinnvolle Ausgestaltung der Erfindung sieht vor, dass das Bildmaterial zur Simulation einer Laufumgebung und/oder zur bildlichen Darstellung von Anforderungen, Fragen oder dergleichen auf die gang-wirksam strukturierte Oberfläche des Endlosbandes abgestimmt ist und das Film- oder Video-Wiedergabegerät und das Laufband zur Gewährleistung einer zeitlichen Abstimmung durch eine Oberflächen/Bild-Synchronisationsstufe synchronisiert sind. Noch spezieller kann vorgesehen sein, dass die Oberflächen/Bild-Synchronisationsstufe und die Synchronisationsstufe zur Synchronisation der Verarbeitung der Druckverteilungsbilder derart abgestimmt programmierbar sind, dass bei der Verarbeitung der Druckverteilungsbilder sowohl die aktuelle gang-wirksamen Oberflächenelemente der Lauffläche als auch hierauf abgestimmte Bildinhalte einbezogen sind, die dem Probanden beim Gehen bzw. Laufen dargestellt werden.

Für eine überzeugende Simulation natürlicher Laufumgebungen und zur Erzielung verwertbarer Ergebnisse ist es wichtig, dass die Simulations-Darstellung (Schirmprojektion) hinreichend genau auf die aktuelle Laufbandgeschwindigkeit abgestimmt ist und insbesondere eine mit steigender Übungsdauer anwachsende Zeitverschiebung vermieden wird. Dies kann zum einen durch manuelle Eingabe einer vorgewählten Laufbandgeschwindigkeit und Konstanthaltung sowohl der Laufbahnwie auch der Anzeigegeschwindigkeit erreicht werden. Flexibler ist jedoch eine Lösung, bei der die aktuelle Laufbandgeschwindigkeit laufend erfasst und die Anzeigegeschwindigkeit im Ansprechen hierauf gesteuert wird.

Die Bandgeschwindigkeit kann über eine Messung der Drehgeschwindigkeit der das Band antreibenden Walze oder über die Erfassung der Fortpflanzungsgeschwindigkeit bestimmter speziell vorgesehener Muster (Kodierungen) auf der Bandunter- oder Bandoberseite erfolgen, oder auch durch Mustererkennung der durch den Benutzer erzeugten Druckverteilungsbilder auf der Drucksensormatrix, wie in der früheren Patentanmeldung PCT/EP2006/010471 genauer beschrieben.

Den oben skizzierten Vorrichtungsaspekten der Erfindung entsprechen in für den Fachmann ohne weiteres ersichtlicher Weise zugleich Verfahrensaspekte, so dass eine gesonderte Auflistung verzichtbar ist. Auch ansonsten ist die Ausführung der Erfindung nicht auf die oben konkret angeführten Aspekte und Ausführungsformen beschränkt, sondern ebenso in einer Vielzahl von Abwandlungen möglich, die im Rahmen fachgemäßen Handelns liegen. Vorteile und Zweckmäßigkeiten der Erfindung ergeben sich aus der nachfolgenden Beschreibung einiger Ausführungsbeispiele anhand der Figuren. Von diesen zeigen:
Fig.1 eine skizzenartige Darstellung einer beispielhaften Gesamtanordnung der erfindungsgemäßen Art,
Fig. 2 und 3 skizzenhafte Darstellungen von Ausgestaltungen des Laufbandes zur Realisierung einer wirksam strukturierten Oberfläche und
Fig. 4 eine skizzenhafte Darstellung eines für einen Benutzer der Anordnung projizierten Schirmbildes.

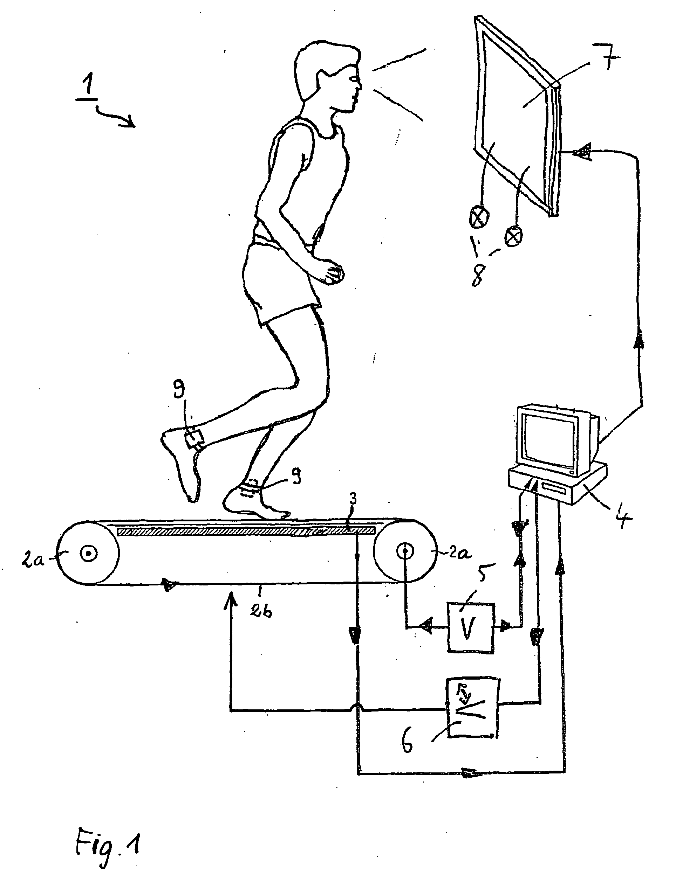

Fig. 1 zeigt ein Laufband-Trainingssystem 1, welches ein über zwei Walzen 2a laufendes Laufband 2b umfasst, unter dessen oberer, vom Benutzer genutzten Oberfläche eine räumlich hochauflösende Druckerfassungsplatte 3 mit einer Vielzahl von (nicht einzeln bezeichneten) matrixförmig angeordneten Drucksensoren zur Erfassung von durch den Benutzer beim Auftreten auf das Laufband erzeugten Druckerfassungsbildern vorgesehen ist. Die eine der beiden Laufrollen 2a ist angetrieben und zieht das Band 2b mit einer vorbestimmten Geschwindigkeit, die über eine Verarbeitungs- und Steuereinheit 4 der Anordnung und einen Geschwindigkeitsregler 5 eingestellt wird. Als weiterer wesentlicher Betriebsparameter der Anordnung kann (was in der Figur lediglich symbolisch dargestellt ist) über ein geeignetes Neigungs-Stellglied 6, welches ebenfalls Störsignale von der Verarbeitungs- und Steuereinheit 4 empfangen kann, eine Neigung des Laufbandes bedarfsgerecht eingestellt werden.

Bei der in Fig. 1 dargestellten, stark vereinfachten Ausführung gelangen den eingestellten Geschwindigkeitswert des Laufbandes charakterisierende Signale vom Geschwindigkeitsregler 5 zurück zur Verarbeitungs- und Steuereinheit 4 und dienen dort zur Synchronisation einer bildlichen Darstellung auf einem Anzeigeschirm 7, der im Blickfeld des Benutzers platziert ist und diesem eine natürliche Laufstrecke simulieren kann. Die bildliche Darstellung wird anhand der Geschwindigkeitssignale derart gesteuert, dass - insbesondere in Verbindung mit einer weiter unten beschriebenen speziellen Ausgestaltung - dem Nutzer eine insgesamt stimmige Simulation einer Laufumgebung, vorteilhaft verknüpft mit der Simulation von zu überwindenden oder zu vermeidenden Hindernissen, präsentiert wird. Abweichend von der Darstellung in der Figur, kann auch die tatsächliche Geschwindigkeit des Laufbandes über eine geeignete (nicht dargestellte) Sensorik erfasst und der Messwert der Verarbeitungs- und Steuereinheit 4 zum gleichen Zweck zugeführt werden.

In einer besonderen Ausführungsform der Geschwindigkeitseinstellung kann berücksichtigt werden, dass ein Mensch selbst bei gleichförmigen Gehen keine vollständig konstante Momentangeschwindigkeit einhält. Mit der dargestellten Anordnung kann im Hinblick hierauf die Geschwindigkeit des Bandes wahlweise sogar bei jedem Schritt, abhängig vom Abrollmuster der Füße, welches durch die Druckerfassungsplatte 3 erfasst wird, etwas variiert werden. So kann während der Abrollphase des linken und rechten Fußes die Bandgeschwindigkeit jeweils etwas vermindert oder erhöht werden. Diese leichten Variationen der Bandgeschwindigkeit geben dem Benutzer Rückkopplungen, welche ihn zu einem natürlicheren Gehen auf dem Laufband anregen.

Die Drucksensoren der Druckerfassungsplatte können wahlweise eine analoge oder - in einer vereinfachten und kostengünstigeren Ausführung - eine digitale Ansprechcharakteristik (Aus/Ein-Charakteristik) haben. Beide Varianten haben für bestimmte Anwendungen ihre Berechtigung, und der Auswahl einer der Varianten wird der Systementwerfer nach den primären Einsatzanforderungen treffen.

Anstelle des in Fig. 1 schematisch dargestellten Bildschirms 7 kann auch eine Anordnung aus mehreren Bildschirmen, die den Benutzer teilweise umgeben können, oder eine Video-Projektionseinrichtung (Laserbeamer etc.) vorgesehen sein. Wie in der Figur dargestellt, kann die zur Simulation dienende Anzeigefläche um Hinweis- bzw. Warn-Anzeigeelemente 8 ergänzt sein, mit denen der Nutzer zu bestimmten Handlungen oder Unterlassungen aufgefordert wird. Mit derartigen Anzeigeelementen kann er beispielsweise aufgefordert werden, eine definierte Fläche des Laufbandes oder einen hervorgehobenen Flächenbereich in der Simulation der Laufumgebung bewusst zu betreten oder aber zu meiden. In Verbindung hiermit können dem Benutzer über derartige Anzeigeelemente dann auch unmittelbar Rückmeldungen über den Erfolg seines Bemühens gegeben werden. Diese Anzeigeelemente können teilweise auch akustische Anzeigen realisieren oder mit solchen kombiniert sein.

Zur Durchführung von Trainigsaufgaben am Laufband-System kann es von Interesse sein, die Höhe des Abhebens der Füße vom Band zu erkennen z.B. dann, wenn der Proband ein virtuelles Hindernis übersteigen soll. Deshalb hat der Proband in einer weiteren Ausführungsform mindestens einen Sensor 9 an einem Fuß befestigt, dessen Signale mittels einer an sich bekannten (hier nicht dargestellten) Positionserfassungs-Sensorik erfasst werden können, um Rückschlüsse über die Lage bzw. die Höhe der Füße zu geben. Die Sensoren arbeiten vorzugsweise zeitsynchron mit den Sensoren der Druckverteilungsmatrix. Eine genaue Zeitsynchronisierung kann ggf. über ein Infrarot- oder Funksignal oder über eine Detektion des Zeitpunktes des Auftretens hergestellt werden.

Die Sensoren 9 können als Beschleunigungssensoren oder mehrachsigen Beschleunigungssensoren ausgeführt sein und sind ggf. über Funk mit dem Auswerterechner 4 verbunden. Aus den Beschleunigungssignalen kann die Position der Füße errechnet werden, insbesondere dann, wenn zusätzlich die Zeit- und Ortsabhängigkeit der Druckverteilungsmuster in die Berechnung mit eingehen können. In erweiterten Anordnungen können Inertialsensorsysteme zur Anwendung kommen, bei denen zusätzlich Gyroskope oder Sensoren zur Detektion des Erdmagnetfeldes eingesetzt werden. Solche Sensoren können natürlich auch an anderen Körperabschnitt befestigt werden, sodaß die Bewegung der kompletten unteren Extremitäten oder des ganzen Körpers gemessen und dargestellt werden kann. Die Sensoren 9 können aber auch nach anderen Messprinzipien arbeiten, zum Beispiel auf der Basis aktiver oder passiver Lichtmarker die von stationären Kameras aufgenommen werden, Magnetfeldesensoren oder Sensoren die Ultraschallwellen zu stationären Empfängern abgeben oder von dort empfangen und aus der Laufzeit des Schalls die Position der Füße bestimmen.

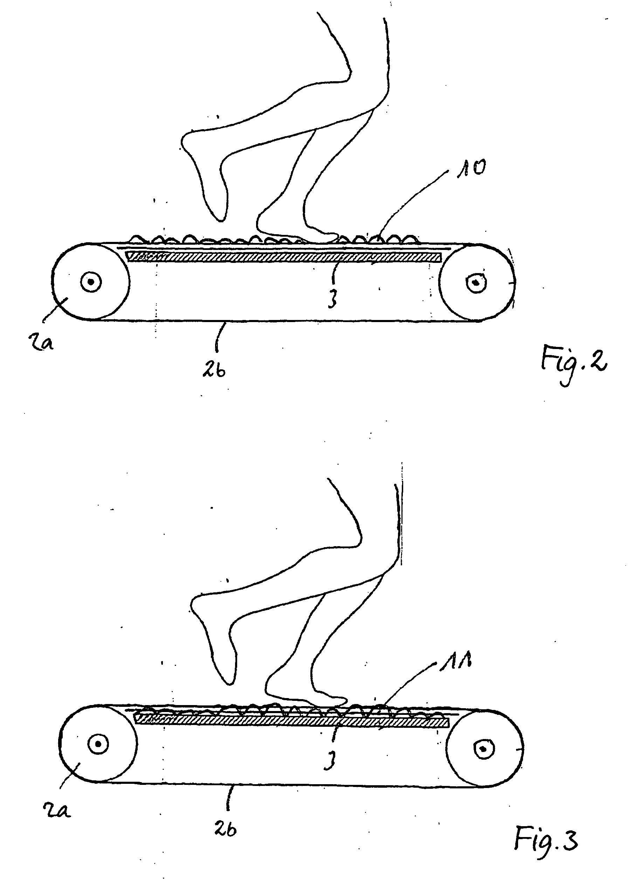

In Fig. 2 ist ein Ausführungsbeispiel dargestellt, bei dem eine strukturierte Oberfläche über Aktuatoren 10 ausgeführt ist. Die Aktuatoren 10 befinden sich im Laufband 2b und bewegen sich über die Rollen des Laufband - Systems 2. Sie werden vorzugsweise aktiviert, wenn diese an der Gangoberfläche erscheinen. Im Ausführungsbeispiel sind flüssigkeits- oder luftgefüllte Kammern dargestellt. Diese können als komplette Zeilen oder als einzelne matrixförmig angeordnete Kammern ausgeführt sein.

In Fig. 3 sind die Aktuatoren 11 unterhalb des Laufbandes 2b angeordnet. Dieses gleitet über die Aktuatoren 11 Wie in Figur 2 und 3 dargestellt, werden die Kräfte über die Aktuatoren auf die Druckverteilungs-Sensormatrix 3 übertragen.

Werden die in Figur 2 oder 3 gezeigten Aktuatoren in das in Figur 1 dargestellte Laufband-Trainingssystem integriert, so können weitgehend natürliche Gehbedingungen simuliert werden. Es können verschiedene Untergrundbeschaffenheiten an der Anzeigeeinheit in der virtuellen Laufumgebung dargestellt werden. Beispielsweise kann ein Stein auf dem Laufweg virtuell dargestellt werden. Der Proband sieht den Stein an der Anzeigeeinheit zusammen mit seinen Fußabdrücken, kann diesem ausweichen oder fühlt den Stein über die Aktuatoren, wenn er darauf tritt. In der Rehabilitation ermöglicht diese erfindungsgemäße Anordnung, daß der Patient sich an das Gehen in natürlicher Umgebung gewöhnen bzw. das Gehen in natürlicher Umgebung trainieren kann ohne daß er Gefahr läuft zu stürzen.

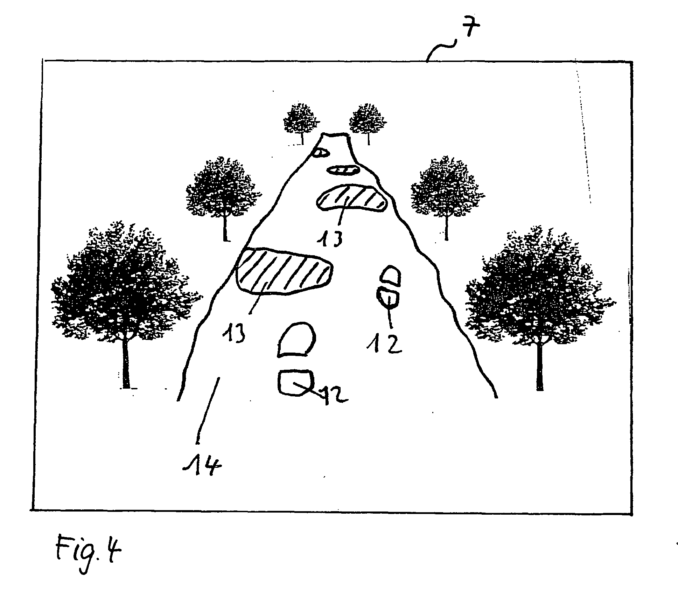

In Fig. 4 ist ein Ausführungsbeispiel einer virtuellen Laufumgebung dargestellt, wie sie in der Anzeigeeinheit 7 zur Darstellung kommen kann. Auf einem virtuellen Laufweg 14 sind Abdrücke 12 der Druckverteilungsmesssensoren 3 sichtbar. Diese bewegen sich vorzugsweise synchron mit den tatsächlichen Fußabdrücken des Probanden am Laufband. In einer bevorzugten Ausführungsform bewegt sich der Laufweg 14 in einer mit der Laufbandgeschwindigkeit synchronisierten Geschwindigkeit.

Die Fußabdrücke 13 können als Umrisslinien, als künstliche Sohlenabdrücke als tatsächliche Druckverteilungsbilder sowohl zwei- als auch dreidimensional dargestellt werden. Gegebenenfalls können auch dreidimensionale Modelle in Form von Füßen oder Schuhen oder jeder anderer Ausbildungsform zur Darstellung kommen. Möglich ist es auch, mit den Füßen am Laufband virtuelle Gegenstände auf Rädern, wie ein Fahrrad oder Auto, oder einen Gegenstand auf Kufen zu steuern.

Auf dem Laufweg 14 befinden sich in der Darstellung der Fig. 4 virtuelle Bereiche 13, die nicht betreten werden dürfen. Diese können beispielsweise als Wasserpfützen dargestellt sein. Es können auch andere Flächen oder auch räumliche Objekte, wie Vertiefungen, Felsen, Hindernisse u.a. gezeigt (simuliert) werden.

In einer weiteren Ausgestaltung sollen die Bereiche 13 betreten und die umliegenden Bereiche des Laufwegs 14 nicht betreten werden. Die Bereiche 13 können sowohl nach dem Zufallsprinzip und in unterschiedlicher Ausformung auftauchen als auch in gleicher Ausformung und/oder in regelmäßigen Abständen. Gegebenenfalls kann auch zur Aufgabe gestellt werden, den Aufpressdruck der Füße am Laufband zu verändern, oder es kann die Laufbandgeschwindikeit variiert werden, wenn z.B. der Aufpressdruck höher oder niedriger ist. In gleicher Weise kann die Simulation der Laufumgebung gesteuert werden, wenn die Druckverteilung am Laufband von einem normalen Abrollvorgang abweicht.

Zur Überprüfung des Erfolges der gestellten Aufgaben wird erfindungsgemäß vom System ein Feedbacksignal zurückgemeldet. Im Ausführungsbeispiel nach Figur 4 kann dies dadurch geschehen, daß sich die Wasserfläche verfärbt oder bewegt wenn in die virtuelle Pfütze getreten wird. Gegebenenfalls können zur Erfolgskontrolle Punkte auf ein Punktekonto gutgeschrieben oder abgezogen werden. Zur Rückkopplung des Erfolges bei der Bewältigung der gestellten Aufgabe können im weiteren die verschiedensten Möglichkeiten des visuellen oder akustischen Feedbacks zur Anwendung kommen.

In einer bevorzugten Ausführungsform werden im Bereich der Rehabilitation dem Patienten mit Gangstörungen virtuell Bereiche vorgegeben, auf die er seine Füße Schritt für Schritt setzen soll. Das Laufband-Trainingssystem überprüft die tatsächlich gemachten Schritte und kann über ein akustisches oder visuelles Feedback, evtl. sogar über eine Sprachausgabe, Informationen geben, ob die Schritte richtig ausgeführt worden sind oder was besser gemacht werden kann.

Bevorzugt werden die Druckverteilungs-Messwerte der am Laufband durchgeführten Schritte jeder Abrollphase am Auswerterechner 4 zwischengespeichert. Damit können die Druckverteilungsbilder bereits während oder nach einer Messung analysiert und für diagnostische Zwecke oder zur Erstellung von orthopädischen Einlegesohlen verwendet werden.

Bei diesen und ähnlichen Anwendungen ist von besonderem Vorteil, daß durch die gestellten Aufgaben eine größere Variabilität der Druckverteilungsbilder vorhanden ist als dies bei einem sonstigen "eingeschliffenen" Gang auf dem Laufband zustande käme. Eine besondere Anwendung ergibt sich bei der Sturzprävention bei älteren Menschen. Es ist bekannt, daß die Sturzgefahr beim Gehen bei älteren Menschen zunimmt, wenn diese zwei Aufgaben gleichzeitig erledigen müssen. Um diese Sturzgefahr zu testen, wird auf dem virtuellen Laufweg für den Probanden überraschend eine weitere Aufgabe eingeführt, die es zu bewältigen gilt. Beispielsweise können virtuelle Gegenstände oder Gefahren auftauchen. Aus der Analyse der dynamischen Druckverteilungsbilder können dann Veränderungen der Gangsicherheit abgeleitet werden.

## Patentansprüche

1. Anordnung zur Ganganalyse (1), insbesondere zu Trainings- oder Rehabilitationszwecken, mit
einem über mindestes zwei Laufrollen (2a) geführten und als Laufband dienenden Endlosband (2b), dessen eine Oberfläche als Lauffläche dient, einer Sensorik (3) zur Bestimmung einer Druck-/Kraftverteilung auf einer unterhalb der Lauffläche befindlichen Kraftmessplatte, die auf der dem Endlosband (2b) zugewandten Seite eine Vielzahl von Druck-/Kraftsensoren aufweist,
einer mit den Druck-/Kraftsensoren eingangsseitig verbundenen Auswertungseinheit (4), welche eingerichtet ist Druckverteilungen zu detektieren, die durch einen gehenden oder laufenden Probanden auf der Lauffläche erzeugt werden, und
einer eingangsseitig mit der Auswertungseinheit (4) verbundenen Verarbeitungseinheit (4), welche eingerichtet ist aus den Druckverteilungen Werte bzw. Signale zu erzeugen, die den Gang des Probanden charakterisieren,
einer dem Laufband zugeordneten, im Sichtfeld des Probanden angeordneten Bildanzeigefläche,
einem Film- oder Video-Wiedergabegerät (7)mit in einem Bildmaterialspeicher gespeichertem Bildmaterial oder einem Anschluss zur Verbindung mit einer Bildmaterial-Datenbasis zum Empfang von Bildmaterial zur Simulation einer Laufumgebung und/oder zur bildlichen Darstellung von Anforderungen, Fragen oder dergleichen an den Probanden sowie
einer Synchronisationsstufe (4) zur Synchronisation der Verarbeitung der Druckverteilungsbilder mit den bildlichen Darstellungen auf der Bildanzeigefläche.

2. Anordnung nach Anspruch 1, wobei das Film- oder Video-Wiedergabegerät (7) durch einen PC oder Fernsehgerät gebildet ist, dessen Bildschirm als Bildanzeigefläche dient.

3. Anordnung nach Anspruch 1, wobei das Film- oder Video-Wiedergabegerät (7) einen Laserbeamer aufweist und die Bildanzeigefläche als Projektionsfläche ausgebildet ist.

4. Anordnung zur Ganganalyse nach einem der Ansprüche 1-3,
wobei das Endlosband (2b) eine, insbesondere unregelmäßig, gang-wirksam strukturierte Oberfläche hat und/oder unter der Lauffläche eine Anordnung von auf das Endlosband einwirkenden Aktoren (10,11) zur lokalen Verformung der Bandoberfläche zur Erzeugung von Unebenheiten der Lauffläche vorgesehen ist.

5. Anordnung nach Anspruch 4, wobei
das Endlosband (2b) mit gang-wirksam strukturierter Oberfläche eine Positionskodierung zur Positionszuordnung von Oberflächenelementen aufweist, die Verarbeitungseinheit (4) Profilspeichermittel zur Speicherung des Profils der gang-wirksam strukturierten Oberfläche aufweist und ihr ein Positionssignalempfänger zur Adressierung des Profilspeichers zugeordnet ist und in der Verarbeitungseinheit (4) ein Verarbeitungsalgorithmus implementiert ist, mit dem die Druckverteilungsbilder den Oberflächenelementen der Lauffläche zugeordnet werden.

6. Anordnung nach Anspruch 4, wobei
der Anordnung von Aktoren (10,11) eine Profilsteuereinheit zugeordnet ist, die Steuersignale zur Betätigung der Aktoren (10,11) zur Ausbildung eines vorbestimmten dynamischen Profils der Lauffläche ausgibt, und
die Verarbeitungseinheit (4) einen Steuersignalempfänger zum Empfang der durch die Profilsteuereinheit ausgegebenen Steuersignale als Positionszuordnungssignale aufweist und
in der Verarbeitungseinheit (4) ein Verarbeitungsalgorithmus implementiert ist, mit dem die Druckverteilungsbilder dem dynamischen Profil der Lauffläche zugeordnet werden.

7. Anordnung nach einem der Ansprüche 4 bis 6, wobei die Anordnung von Aktoren (10,11) eine Vielzahl von einzeln angetriebenen Stößeln zur Erzeugung von Erhebungen auf der Lauffläche aufweist.

8. Anordnung nach Anspruch 4 oder 5, wobei im Endlosband (2b) oder unterhalb der Lauffläche eine Anordnung von jeweils einen steuerbaren Fluidanschluss aufweisenden, durch Fluiddruck elastisch aufweitbaren Kammern zur Ausbildung von Erhebungen auf der Lauffläche vorgesehen ist.

9. Anordnung nach einem der vorangehenden Ansprüche, mit Neigungseinstellmitteln (6) zur Neigungseinstellung und/oder Geschwindigkeitseinstellmitteln (5) zur Einstellung des Laufgeschwindigkeit des Laufbandes.

10. Anordnung nach Anspruch 9, wobei die Neigungseinstellmittel (6)und/oder Geschwindigkeitseinstellmittel (5) mit dem Film- oder Video-Wiedergabegerät (7) synchronisiert sind, derart, dass die Neigungs- bzw. Geschwindigkeitseinstellung in Abstimmung auf ein dem Probanden dargestelltes Bildmaterial und/oder von Anforderungen oder Fragen an den Probanden ausführbar ist.

11. Anordnung nach einem der Ansprüche 1 bis 3, 9 oder 10, wobei die Synchronisationsstufe (4) mit sich aus dem Bildmaterial oder Anforderungen, Fragen oder dergleichen an den Probanden ergebenden Daten programmierbar ist, derart, dass die Verarbeitung der Druckverteilungsbilder anhand entsprechender Vorgaben erfolgen kann.

12. Anordnung nach einem der vorangehenden Ansprüche, mit zusätzlichen Messmitteln (9) zur Erfassung mindestens einer zweiten biometrischen oder einer medizinischen Messgröße, wobei die Verarbeitungseinheit zur kombinierten Verarbeitung der Druckverteilungsbilder und von Messwerten der biometrischen bzw. medizinischen Messgröße ausgebildet ist.

13. Anordnung nach Anspruch 12, wobei eine Synchronisationseinheit (4) zur Synchronisation der Druck-/Kraftverteilungsmessung mit der Erfassung der oder einer weiteren biometrischen bzw. medizinischen Messgröße durch die zusätzlichen Messmittel (9) vorgesehen ist.

14. Anordnung nach Anspruch 13, wobei die Synchronisationseinheit (4) eingangsseitig mit der Auswertungs- oder Verarbeitungseinheit (4) verbunden ist, derart, dass Synchronisationssignale im Ansprechen auf Merkmale der Druckverteilungen oder auf die daraus abgeleiteten Werte bzw. Signale ausgegeben werden.

15. Anordnung nach einem der Ansprüche 1 bis 3 und einem der Ansprüche 4 bis 14, wobei das Bildmaterial zur Simulation einer Laufumgebung und/oder zur bildlichen Darstellung von Anforderungen, Fragen oder dergleichen auf die gang-wirksam strukturierte Oberfläche des Endlosbandes (2b) abgestimmt ist und das Film- oder Video-Wiedergabegerät und das Laufband zur Gewährleistung einer zeitlichen Abstimmung durch eine Oberflächen/Bild-Synchronisationsstufe (4) synchronisiert sind.

16. Anordnung nach Anspruch 15, wobei die Oberflächen/Bild-Synchronisationsstufe (4) und die Synchronisationsstufe (4) zur Synchronisation der Verarbeitung der Druckverteilungsbilder derart abgestimmt programmierbar sind, dass bei der Verarbeitung der Druckverteilungsbilder sowohl die aktuellen gang-wirksamen Oberflächenelemente der Lauffläche als auch hierauf abgestimmte Bildinhalte einbezogen sind, die dem Probanden beim Gehen bzw. Laufen dargestellt werden.

## Claims

1. A gait analysis arrangement (1), in particular for training or rehabilitation purposes, comprising
an endless belt (2b) guided over at least two rollers (2a) and serving as a treadmill, one surface thereof serving as a running surface,
a sensor system (3) for determining a pressure/force distribution across a force-measuring plate situated below the running surface which comprises a plurality of pressure/force sensors on the side facing the endless belt (2b),
an evaluation unit (4) connected on the input side to the pressure/force sensors which is adapted to detect pressure distributions generated across the running surface by a walking or running test person, and
a processing unit (4) connected on the input side to the evaluation unit (4) which is adapted to generate values or signals from the pressure distributions which characterize the test person's gait,
an image display surface arranged in the test person's field of view to face the treadmill,
a film or video reproducing device (7) having visual material stored in a visual material memory or a port to connect to a visual material data base to receive visual material to simulate a running environment and/or to visually display requirements, questions or the like to the test person, as well as a synchronization stage (4) to synchronize the processing of the pressure distribution images with the visual representations on the image display surface.

2. The arrangement according to claim 1, wherein the film or video reproducing device (7) is constituted by a PC or a TV set, the screen of which serves as an image display surface.

3. The arrangement according to claim 1, wherein the film or video reproducing device (7) has a laser projector and the image display surface is configured as a projection surface.

4. The gait analysis arrangement according to any one of claims 1 to 3,
wherein the endless belt (2b) has a particularly irregular, gait-operative structured surface and/or an arrangement of actuators (10, 11) acting on the endless belt is provided below the running surface for locally deforming the upper belt surface so as to generate irregularities in the running surface.

5. The arrangement according to claim 4, wherein
the endless belt (2b) having the gait-operative structured surface comprises a position coding for the position allocating of surface elements, the processing unit (4) comprises profile storing means for storing the profile of the gait-operative structured upper surface and has an associated position signal receiver for addressing the profile storage, and a processing algorithm is implemented in the processing unit (4) by means of which the pressure distribution images are associated with the surface elements of the running surface.

6. The arrangement according to claim 4, wherein
the arrangement of actuators (10, 11) has an associated profile control unit which outputs control signals for operating the actuators (10, 11) so as to form a predefined dynamic profile of the running surface, and the processing unit (4) comprises a control signal receiver for receiving the control signals output by the profile control unit as position-allocating signals, and a processing algorithm is implemented in the processing unit (4) by means of which the pressure distribution images are associated with dynamic profile of the running surface.

7. The arrangement according to any one of claims 4 to 6, wherein the arrangement of actuators (10, 11) comprises a plurality of individually driven tappets for generating elevations on the running surface.

8. The arrangement according to claim 4 or 5, wherein an arrangement of chambers each having a controllable fluid connection and being elastically expandable by fluid pressure is provided in the endless belt (2b) or below the running surface to form elevations on the running surface.

9. The arrangement according to any one of the preceding claims, having inclination adjusting means (6) for inclination adjustment and/or speed adjusting means (5) for adjusting the running speed of the treadmill.

10. The arrangement according to claim 9, wherein the inclination adjusting means (6) and/or the speed adjusting means (5) are synchronized with the film or video reproducing device such that the inclination or speed adjusting can be performed based on visual material presented to the test person and/or requirements or questions to the test person.

11. The arrangement according to any one of claims 1 to 3, 9 or 10, wherein the synchronization stage (4) can be programmed using data resulting from the visual material or requirements, questions or the like to the test person such that the pressure distribution images can be processed on the basis of the corresponding input.

12. The arrangement according to any one of the preceding claims, having additional measuring means (9) for detecting at least one second biometric or medical parameter, wherein the processing unit is configured for a combined processing of the pressure distribution images and measured biometric or medical parameter values.

13. The arrangement according to claim 12, wherein the synchronization unit (4) is provided to synchronize the pressure/force distribution measurement with the detecting of the one or a further biometric or medical parameter by the additional measuring means (9).

14. The arrangement according to claim 13, wherein the synchronization unit (4) is connected on the input side to the evaluation or processing unit (4) such that synchronization signals are output in response to characteristics of the pressure distributions or the values or signals derived form same.

15. The arrangement according to any one of claims 1 to 3 and any one of claims 4 to 14, wherein the visual material for simulating a running environment and/or for imaging requirements, questions or the like is matched to the gait-operative structured surface of the endless belt (2b), and the film or video reproducing device and the treadmill are synchronized by a surface/image synchronization stage (4) to ensure coordination over time.

16. The arrangement according to claim 15, wherein the surface/image synchronization stage (4) and the synchronization stage (4) for synchronizing the processing of the pressure distribution images can be programmed in such a coordinated manner that the processing of the pressure distribution images includes both the current gait-operative surface elements of the running surface and the visual content matched to same as presented to the test person while walking or running.

## Revendications

1. Montage pour l'analyse de la marche (1), en particulier à des fins d'entraînement ou de rééducation, comportant
une bande sans fin (2b) guidée via au moins deux rouleaux de roulement (2a) et servant de tapis de course et dont une surface sert de surface de course,
un dispositif de capteurs (3) destiné à déterminer une répartition de pression / de force sur une plaque de mesure de force située en-dessous de la surface de course et qui présente une pluralité de capteurs de pression / de force du côté tourné vers la bande sans fin (2b),
une unité d'évaluation (4) reliée côté entrée aux capteurs de pression /de force et qui est configurée pour détecter des répartitions de pression qui sont générées sur la surface de course par un sujet d'expérimentation qui marche ou qui court, et
une unité de traitement (4) reliée côté entrée à l'unité d'évaluation (4) et qui est configurée pour générer, à partir des répartitions de pression, des valeurs et/ou signaux qui caractérisent la marche du sujet d'expérimentation,
un panneau d'affichage d'images associé au tapis de course et disposé dans le champ visuel du sujet d'expérimentation,
un appareil de restitution de film ou de vidéo (7) avec du matériel d'imagerie enregistré dans une mémoire de matériel d'imagerie ou un raccord destiné à assurer la liaison avec une base de données de matériel d'imagerie en vue de la réception de matériel d'imagerie pour la simulation d'un environnement de course et/ou pour la représentation imagée de demandes, questions ou similaires au sujet d'expérimentation ainsi que
un étage de synchronisation (4) destiné à synchroniser le traitement des images de répartition de pression avec les représentations imagées sur le panneau d'affichage d'images.

2. Montage selon la revendication 1, où l'appareil de restitution de film ou de vidéo (7) est constitué par un PC ou un appareil de télévision dont l'écran sert de panneau d'affichage d'images.

3. Montage selon la revendication 1, où l'appareil de restitution de film ou de vidéo (7) présente un vidéoprojecteur laser et le panneau d'affichage d'images est constitué comme surface de projection.

4. Montage pour l'analyse de la marche selon l'une des revendications 1 à 3, où la bande sans fin (2b) a une surface structurée de façon efficace pour la marche, en particulier de façon irrégulière, et/ou un montage d'actionneurs (10, 11) agissant sur la bande sans fin étant prévu sous la surface de course pour déformer localement la surface de bande afin de générer des inégalités de la surface de course.

5. Montage selon la revendication 4, où
la bande sans fin (2b) à surface structurée de façon efficace pour la marche présente un codage de position pour l'attribution de position d'éléments de surface, l'unité de traitement (4) présente des moyens d'enregistrement de profil pour l'enregistrement du profil de la surface structurée de façon efficace pour la marche et un récepteur de signal de position lui est attribué pour l'adressage de la mémoire de profil et un algorithme de traitement avec lequel les images de répartition de pression sont attribuées aux éléments de surface de la surface de course est implémenté dans l'unité de traitement (4).

6. Montage selon la revendication 4, où
une unité de commande de profil qui émet des signaux de commande pour l'actionnement des actionneurs (10, 11) en vue de constituer un profil dynamique prédéterminé de la surface de course est attribuée au montage d'actionneurs (10, 11), et
l'unité de traitement (4) présente un récepteur de signaux de commande destiné à recevoir les signaux de commande émis par l'unité de commande de profil comme signaux d'attribution de position, et
un algorithme de traitement avec lequel les images de répartition de pression sont attribuées au profil dynamique de la surface de course est implémenté dans l'unité de traitement (4).

7. Montage selon l'une des revendications 4 à 6, où le montage d'actionneurs (10, 11) présente une pluralité de poussoirs à entraînement individuel destinés à générer des élévations sur la surface de course.

8. Montage selon la revendication 4 ou 5, où un montage de chambres présentant chacune un raccord de fluide pilotable, extensibles élastiquement par pression du fluide pour constituer des élévations sur la surface de course, est prévu dans la bande sans fin (2b) ou sous la surface de course.

9. Montage selon l'une des revendications précédentes, comportant des moyens de réglage d'inclinaison (6) pour le réglage d'inclinaison et/ou des moyens de réglage de vitesse (5) pour le réglage de la vitesse de course de la bande de course.

10. Montage selon la revendication 9, où les moyens de réglage d'inclinaison (6) et/ou les moyens de réglage de vitesse (5) sont synchronisés avec l'appareil de restitution de film ou de vidéo (7) de telle façon que le réglage d'inclinaison / de vitesse est réalisable en concordance avec un matériel d'imagerie présenté au sujet d'expérimentation et/ou avec des demandes ou questions au sujet d'expérimentation.

11. Montage selon l'une des revendications 1 à 3, 9 ou 10, où l'étage de synchronisation (4) est programmable avec des données résultant du matériel d'imagerie ou des demandes, questions ou similaires au sujet d'expérimentation de telle façon que le traitement des images de répartition de pression peut être effectué à l'aide de spécifications en ce sens.

12. Montage selon l'une des revendications précédentes, comportant des moyens de mesure supplémentaires (9) pour l'acquisition d'au moins une deuxième grandeur de mesure biométrique ou d'une grandeur de mesure médicale, l'unité de traitement étant configurée pour le traitement combiné des images de répartition de pression et de valeurs de mesure de la grandeur de mesure biométrique / médicale.

13. Montage selon la revendication 12, où une unité de synchronisation (4) pour la synchronisation de la mesure de répartition de pression / de force avec l'acquisition de la grandeur de mesure biométrique ou médicale ou d'une grandeur de mesure biométrique ou médicale supplémentaire par les moyens de mesure supplémentaires (9) est prévue.

14. Montage selon la revendication 13, où l'unité de synchronisation (4) est reliée côté entrée à l'unité d'évaluation ou de traitement (4) de telle façon que des signaux de synchronisation sont émis en réponse à des caractéristiques des répartitions de pression ou aux valeurs ou signaux qui en sont dérivés.

15. Montage selon l'une des revendications 1 à 3 et l'une des revendications 4 à 14, où le matériel d'imagerie pour la simulation d'un environnement de course et/ou pour la représentation imagée de demandes, questions ou similaires concorde avec la surface structurée de façon efficace pour la marche de la bande sans fin (2b) et l'appareil de restitution de film ou de vidéo et la bande de course sont synchronisées par un étage de synchronisation surface / image (4) afin de garantir une concordance temporelle.

16. Montage selon la revendication 15, où l'étage de synchronisation surface / image (4) et l'étage de synchronisation (4) pour la synchronisation du traitement des images de répartition de pression sont programmables de manière concordante de telle façon que aussi bien les éléments de surface efficaces pour la marche effectifs de la surface de course que des contenus d'image concordant avec ceux-ci et présentés au sujet d'expérimentation lors de la marche ou de la course sont intégrés lors du traitement des images de répartition de pression.
